# EUROPEAN PATENT SPECIFICATION

(11) **EP 2 374 435 B1**
(45) Date of publication and mention of the grant of the patent: **12.11.2014**
(21) Application number: 11167990.8
(22) Date of filing: 17.09.2009
(51) Int. Cl.: A61F 5/451

(54) **Urine receiver and wearing article**
Urinfänger und Kleidungsstück damit
Récepteur d'urine et article vestimentaire

(30) Priority: 19.09.2008 JP 2008241776; 19.09.2008 JP 2008241778; 19.09.2008 JP 2008241779
(43) Date of publication of application: 12.10.2011
(62) Divisional of application: 09814633.5
(73) Proprietor: Unicharm Corporation, Ehime 799-0111 (JP)
(72) Inventor: Wada, Ichiro, Kanonji-shi, Kagawa 769-1602 (JP); Suzuki, Miou, Kanonji-shi, Kagawa 769-1602 (JP); Yamaki, Rumi, Kanonji-shi, Kagawa 769-1602 (JP); Murakami, Hiroko, Kanonji-shi, Kagawa 769-1602 (JP)
(74) Representative: Knights, Rupert

(56) References cited:
- WO-A1-01/10372
- WO-A1-2008/001475
- JP-A- 2008 029 714
- US-A- 5 342 583
- US-A1- 2007 038 194

## Description

### TECHNICAL FIELD

The present invention relates to wearing articles and more particularly to wearing articles such as pants, disposable diapers or incontinent briefs provided with a suction unit for suction of moisture such as urine.

### RELATED ART

Conventionally, wearing articles such as pants provided on their side facing the wearer's body with a urine sensor are known, for example, from JP 2004-41697 A. According to the disclosure of JP 2004-41697A, aurine sensor is sandwiched between a pair of diapers. The urine sensor includes a pair of electrodes extending in the longitudinal direction and, upon electrical connection of the paired electrodes via urine flowing between them, the urine sensor is turned on to detect occurrence of urination.

A prior art absorbent pad having elasticized side flaps is known from WO 01/10372. A prior art automatic stool and urine disposing apparatus is known from US 5 342 583. A prior art urine receiver is known from US 2007/0038194, which is regarded as the closest prior art. A prior art pants type disposable diaper is known from JP 2008029714.

### DISCLOSURE OF THE INVENTION

### PROBLEM TO BE SOLVED BY THE INVENTION

The urine sensor disclosed in JP 2004-41697 A is designed to be held between a pair of diapers and the urine sensor may inevitably be displaced relatively to the diapers, for example, in the course of putting the diapers on the wearer's body or depending on the wearer's posture after the diapers have been put on the wearer's body. Urination can be detected at a high probability so far as the electrodes of the urine sensor are aligned with the urination region of the wearer but the urination detecting accuracy will be lowered if the urine sensor is displaced and, in consequence, the urine sensor will be out of alignment with the urination region of the wearer. However, even if the urine sensor is displaced relatively to the urination region of the wearer, it is impossible to check on the displacement because the urine sensor is held between a pair of diapers.

It is an object of the present invention to provide the wearing article allowing the accuracy of moisture detection by a moisture detecting structure to be improved.

### MEASURE TO SOLVE THE PROBLEM

According to the present invention, there is provided a wearing article as recited in Claim 1.

According to one embodiment of the present invention, the receiver has a longitudinal center line bisecting a length dimension in the transverse direction and the alignment check mark linearly extends in the longitudinal direction along the longitudinal center line.

According to another embodiment of the present invention, the alignment check mark comprises a first segment linearly extending along the longitudinal center line and a second segment having a length dimension in the transverse direction larger than that of the first segment.

According to still another embodiment of the present invention, at least in the crotch region, the receiver is visually recognized through the chassis. At least in the crotch region, the chassis is formed with windows through which the receiver is exposed.

According to yet another embodiment of the present invention, at least in the crotch region, the chassis is formed with windows through which the receiver is exposed.

### EFFECT OF THE INVENTION

According to the present invention, the alignment check mark adapted to be visually recognized is formed on the side of the receiver including the moisture detecting structure facing the wearer's body. Such an arrangement makes it possible to determine whether the receiver lies on a desired position or not. If the receiver is determined to be displaced from the desired position, the receiver can be repositioned so that the moisture detecting structure may be aligned with the desired position and thereby the desired detection accuracy of the moisture detecting structure may be assured.

### BRIEF DESCRIPTION OF THE DRAWINGS

Fig. 1 is an overall diagram schematically illustrating a system according to a first embodiment of the present invention.
Fig. 2 is a plan view of a urine receiver as viewed from its side facing the wearer's body.
Fig. 3 is a sectional view taken along line III-III in Fig. 2.
Fig. 4 is a sectional view taken along line IV-IV in Fig. 2.
Fig. 5 is a plan view of the urine receiver as viewed from its side facing the wearer's garment.
Fig. 6 is a perspective view of the urine receptacle. Fig. 7 is a diagram similar to Fig. 1.

### DETAILED DESCRIPTION OF THE PREFERRED EMBODIMENTS

Details of the present invention will be more fully understood from the description exemplarily on the basis of pants formed of a mesh sheet as a wearing article incorporated with a urine receiver.

### <First Embodiment>

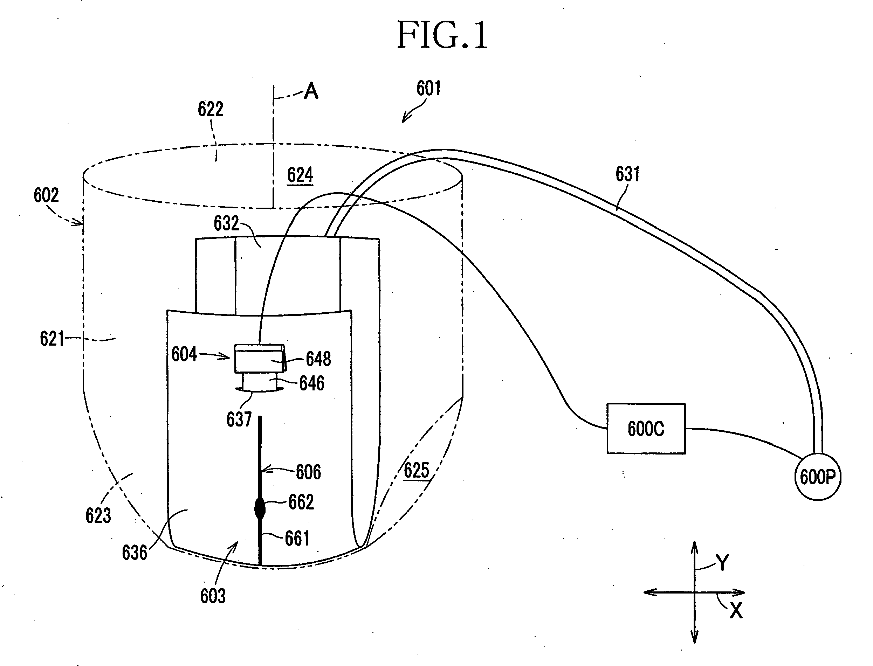

Figs. 1 through 6 illustrate a first embodiment of the present invention.

Fig. 1 is an overall diagram schematically illustrating the wearing article 601 according to the present invention. According to the present invention, a pants-type chassis 602 is formed therein with a urine receiver 603 to which a suction pump 600P as a suction unit via suction tube 631. The urine receiver 603 is electrically connected to the suction pump 600P which is adapted to be actuated by a control unit 600C when a moisture detecting structure 604 incorporated in the urine receiver 603 detects occurrence of urination. Upon actuation of the suction pump 600P, the urine collected by the urine receiver 603 is sucked and then evacuated out from the chassis 602. In Fig. 1, the chassis 602 is indicated by imaginary lines.

The chassis 602 comprises a front waist region 621, a rear waist region 622 and a crotch region 623 extending between these front and rear waist regions 621, 622 wherein these three regions are continuous in the longitudinal direction Y. The chassis 602 is formed of an elastic mesh sheet so that the chassis 602 may be elasticized in the longitudinal direction Y and in the transverse direction X which is orthogonal to the longitudinal direction Y. Particularly along peripheries of a waist-opening 624 and leg-openings 625, the elasticity is locally heightened to improve a fit of the chassis 602 to the wearer's waist and thighs, respectively.

In the chassis 602, the urine receiver 603 can be visually recognized through the elastic mesh formed of elastic yarns.

The receiver 603 extends in the longitudinal direction Y from the front waist region 621 to the rear waist region 622 and along a longitudinal center line A-A bisecting a dimension in the transverse direction X. The receiver 603 is kept in close contact with the wearer' s body under elastic force of the chassis 602 so that the receiver can be independently exchanged with the fresh one.

The receiver 603 includes the topsheet 632 lying on the side facing the wearer's body and the backsheet 636 facing the wearer' s garment wherein the backsheet 636 is formed with a linear alignment check mark 606 extending in the longitudinal direction Y.

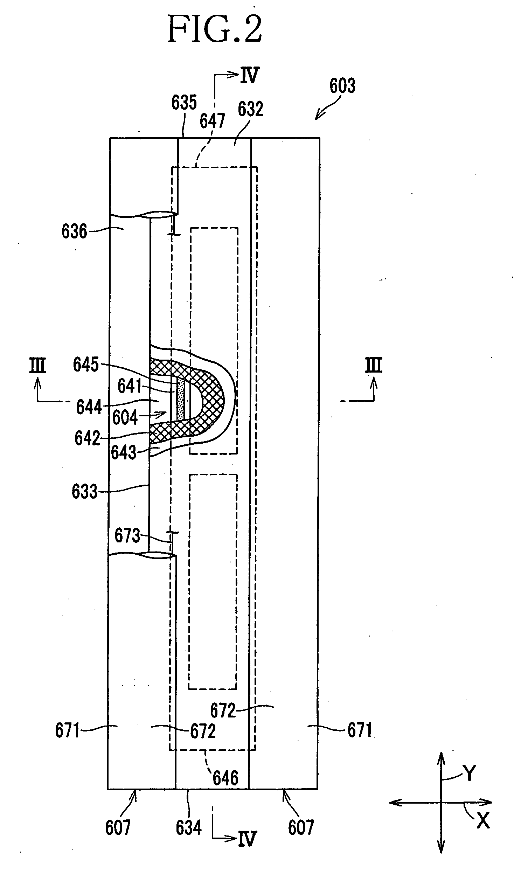

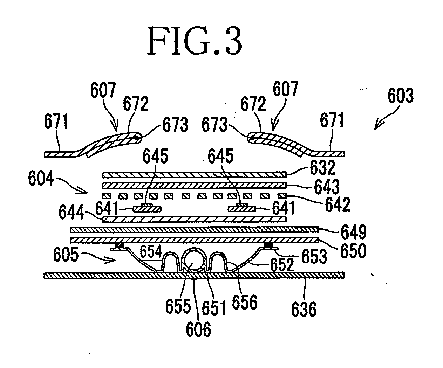

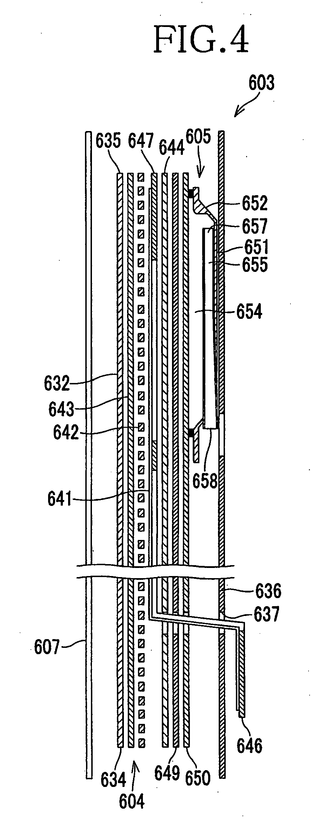

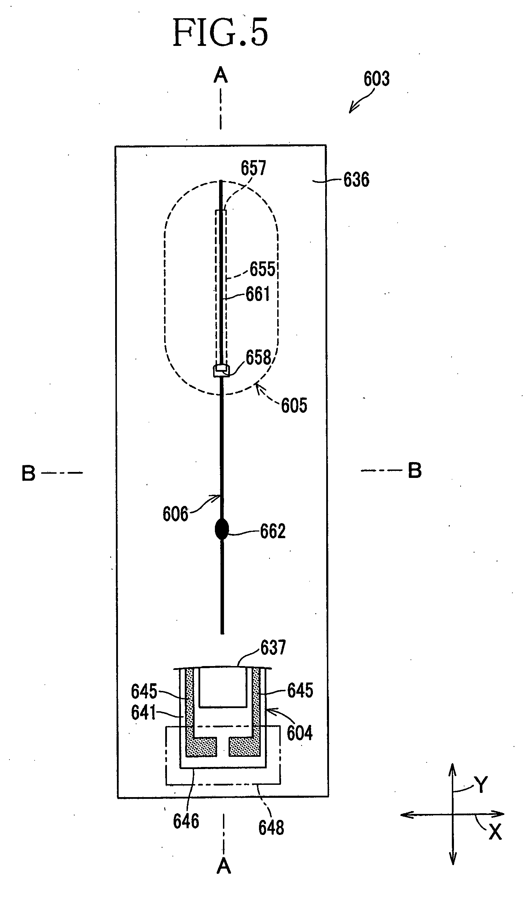

Fig. 2 is a planview showing the receiver 603 in its developed state and as viewed from the side of the wearer's body. In Fig. 2, the receiver 603 is shown as partially cutaway and the chassis 602 is not shown for convenience of explanation. Fig. 3 is a sectional view taken along line XXI-XXI in Fig. 2 and Fig. 4 is a sectional view taken along line XXII-XXII in Fig. 2. In Figs. 3 and 4, the respective sheets are shown as being spaced one from another for convenience of explanation but these sheets are normally joined together. Fig. 5 is a plan view showing the receiver 603 as viewed from the side of the wearer's garment.

A pair of barrier cuffs 607, 607 against body waste is formed on the side of the topsheet 632 facing the wearer's body. The barrier cuffs 607, 607 respectively include proximal edges 671, 671 lying on outer sides as viewed in the transverse direction X and free edges 672, 672 lying on the inner sides as viewed in the transverse direction X. The proximal edges 671, 671 extend outward in the transverse direction X beyond the topsheet 632 and joined to the backsheet 636. The free edges 672, 672 are adapted to be spaced upward from the topsheet 632 toward the wearer's body and provided with cuff elastic elements 673, 673 extending in the longitudinal direction Y and attached thereto under tension and in a contractible manner. Upon contraction of the cuff elastic elements 673, 673, the free edges 672, 672 are spaced upward so as to stand on the topsheet 632 and thereby prevent urine from leaking outward in the transverse direction X. While the liquid-impervious barrier cuffs 607, 607 are effective to prevent leakage of urine, it is possible to use, for example, a moisture-pervious fibrous nonwoven fabric from the viewpoint of permeability for moisture vapor.

Between the top- and backsheets 632, 636, the moisture detecting structure 604 serving to detect occurrence of urination and the tray-shaped urine receptacle 605 lying on the side of the moisture detecting structure 604 facing the wearer's garment and adapted to receive discharged urine.
The moisture detecting structure 604 extends the front waist region 621 to the rear waist region 622 of the chassis 502. The moisture detecting structure 604 comprises a pair of moisture detecting sensors 641, a spacer 642 lying on the side of the moisture detecting sensors 641 facing the wearer's body, a filter 643 lying on the side of the spacer 642 facing the wearer's body and a cushion sheet 644 lying on the side of the moisture detecting sensors 641 facing the wearer's garment. Specifically, the moisture detecting sensors 641 is sandwiched between the spacer 642 and the cushion sheet 644 in the thickness direction and thereby protected from any external shock.

The moisture detecting sensors 641 are formed of a pair of electrodes 645, 645 spaced from each other in the transverse direction X and these electrodes 645, 645 are obtained, for example, by printing a thermoplastic synthetic film such as a polyethylene film with conductive coating material. These electrodes 645, 645 extend in parallel to each other in the longitudinal direction Y. The moisture detecting sensors 641 respectively have front and rear ends 646, 647 extending in the transverse direction X wherein the sensor's front end 646 extends through a slit 637 formed in the backsheet 636 to the side thereof facing the wearer's garment. The slit 637 is formed on the side of the front waist region 621. The sensor's front end 646 extending out through the slit 637 is provided with a clip 648 via which the signal from the moisture detecting sensors 641 is transmitted to the control unit 600C.
More specifically, the moisture detecting sensors 641 are turned on in response to electrical connection of the paired electrodes 645, 645 effectuated by the present of urine therebetween and the control unit 600C detects occurrence of urination on the basis of this turned-on state.

As a material for the spacer 642, a mesh sheet having high air- and liquid-permeability and serves to keep the filter 643 and the moisture detecting sensors 641 spaced from each other in the thickness direction. If the filter 643 in a wet condition continues to be kept in contact with the moisture detecting sensors 641, the moisture detecting sensors 641 will be maintained in the turned-on state and may malfunction to detect urination even though no urination occurs. To prevent such malfunction, the filter 643 and the moisture detecting sensors 641 are kept spaced from each other. For the purpose of preventing such malfunction, use of the spacer is particularly effective since the spacer can effectively prevent these filter 643 and moisture detecting sensors 641 from being continuously kept in contact with each other.

A dispersant sheet 649 and an air permeable-resistant sheet 650 are stacked on the side of the cushion sheet 644 facing the wearer's garment and the urine receptacle 605 is formed on the side of the air permeable-resistant sheet facing the wearer's garment. The air permeable-resistant sheet 650 and the urine receptacle 605 are joined together, for example, by adhesives.
The dispersant sheet 649 serves to disperse the discharged urine as quickly as possible and thereby to change the air permeable-resistant sheet 650 over a wide range thereof into a dry condition. As a material for the air permeable-resistant sheet 649, for example, a nonwoven fabric containing hydrophilic fibers such as rayon fibers may be used.

The air permeable-resistant sheet 650 is high in its liquid-permeability but low in its air permeability and, as a material for this sheet 650, for example, a nonwoven fabric modified to become hydrophilic may be used.

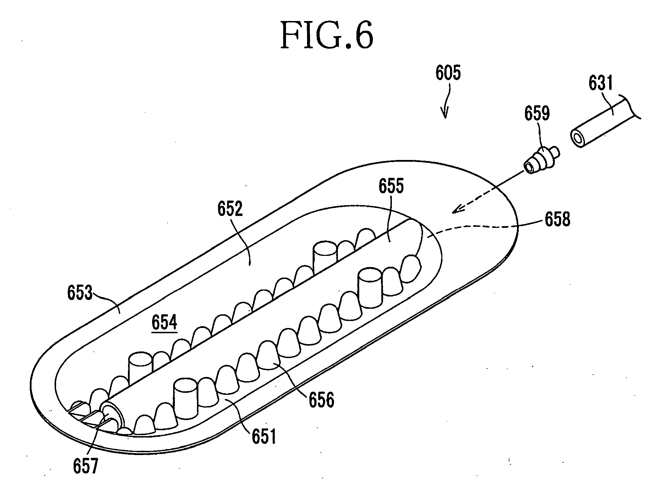

The urine receptacle 605 is flexible and may be formed, for example, by a liquid-impervious soft elastic material such as soft polyethylene or silicon rubber. Fig. 6 is a perspective view of the urine receptacle 605. The urine receptacle 605 comprises a bottom 651, a peripheral wall 652 rising from the bottom 651 toward the side of the wearer's body, and a flange 653 extending outward from a top peripheral edge of the peripheral wall 652. The flange 653 is put in contact with the air permeable-resistant sheet 650 to form a space 654 within the urine receptacle 605. The urine receptacle 605 is formed with a suction unit 655 functioning to collect the urine flowing thereinto and to evacuate this out of the urine receptacle 605. The suction unit 655 has a cylindrical shape and extends along a generally transverse middle zone of the receptacle 605 in the longitudinal direction. On both sides of the suction unit 655, a plurality of protuberances 656 extend upward from the bottom 651 toward the wearer's body. These protuberances 656 serve to prevent the air permeable-resistant sheet 650 which should be held in contact with the flange 653 from sagging down into the space 654.

The suction unit 655 has one end 657 opening into the urine receptacle 605 and the other end 658 opening to the outside of the urine receptacle 605. The suction tube 631 is attached to the other end 658 of the suction unit 655 with a joint 659 and the suction tube 631 is connected to the suction pump 600P.

The backsheet 636 lies on the side of the urine receiver 605 facing the wearer's garment. The backsheet 636 may be formed, for example, of a liquid-impervious fibrous nonwoven fabric. On the side of the backsheet 636 facing the wearer's garment, the alignment check mark 606 is formed (See Figs. 1 and 5). The alignment check mark 606 comprises a linear first segment 661 extending in the longitudinal direction Y and a second segment 662 having a dimension in the transverse direction X larger than that of the first segment 661. The first segment 661 is formed so as to extend along and on the longitudinal center line A-A and the second segment 662 has a generally elliptical shape partially overlapping the first segment 661 and defined on the side of the front waist region 621 about a transverse center line B-B bisecting a dimension of the receiver 603 in the longitudinal direction Y. Such alignment check mark 606 may be formed, for example, by printing the backsheet 636 with appropriate ink or the other coating material.

Now the manner in which the aforementioned arrangement operates in response to occurrence of urination will be described. Upon occurrence of urination, the urine discharged infiltrates into the topsheet 532. The barrier cuffs 507, 507 formed along the side edges 533, 533 of the topsheet prevent the urine from leaking outward in the transverse direction X.
The urine having infiltrated into the topsheet 632 quickly come in contact with the moisture detecting sensors 641 through the spacer 642 and the filter 643. When urine comes in contact with the paired moisture detecting sensors 641 so as to connect these sensors with each other, these moisture detecting sensors 641 are turned on and transmit signals to the control unit 600C. In response to this signal, the control unit 600C actuates the suction pump 600P. Upon actuation of the suction pump 600P, the space 654 of the urine receptacle 605 is subjected to a suction effect via a suction unit 655.

The urine having reached the moisture detecting sensor 641 infiltrates into the dispersant sheet 649 through the cushion sheet 644. The urine having dispersant sheet 649 over a wide range simultaneously infiltrates into the air permeable-resistant sheet 650 which covers the opening of the urine receptacle 605 to define the space 654 therein. When the space 654 is subjected to the suction effect by the suction pump 600P and the opening is covered with the air permeable-resistant sheet 650 wetted with urine, a negative pressure is generated within the space 654. As a result, the urine having infiltrated in the air permeable-resistant sheet 650 is sucked into the space 654 and the urine sucked into the space 654 is sucked through the suction tube 655 from the urine receptacle 605. In this way, immediately after occurrence of urination, the discharged urine can be quickly evacuated out of the wearing article.

In the aforementioned wearing article, the receiver 603, eventually, the alignment check mark 606 formed on the receiver 603 can be visually recognized through the chassis 602. Therefore, it is possible for the care personnel to determine whether the receiver 603 is displaced or not on the basis of the position of the alignment check mark 606 in the course of putting the wearing article 601 on the wearer's body. Occurrence of urination is detected by the moisture detecting sensor 641 when urine comes in contact with a pair of the electrodes 645, 645 so as to bridge these paired electrodes 645, 645. In other words, the receiver 603 may be aligned with the intermediate position between the paired electrodes 645, 645 to achieve the highest detection accuracy.

If the receiver 603 is displaced toward right or left side of the wearer, there is a possibility that the wearer's urine might be discharged outside the paired electrodes 645, 645. In this case, the paired electrodes 645, 645 are bridged by urine only when the urine sufficiently increases to flow beyond one of the electrodes 645 to the other electrode 645. Consequently, it is impossible for a small quantity of urine to bridge the paired electrodes 645, 645 and to be detected. In contrast, if the wearer's urine is discharged just between the paired electrodes 645, 645, it will be easy even for a small quantity of urine to bridge the paired electrodes 645, 645 and to turn them on so that such a small quantity of urine can be reliably detected. In this way, the highest detection accuracy is assured.
According to the invention, the electrodes 645, 645 are formed on both sides of the longitudinal center line A-A and the alignment check mark 606 is formed along the longitudinal center line A-A. With such arrangement, the highest urine detection accuracy can be assured by putting the article on the wearer's body so that the mark 606 may be aligned with the wearer's urethral region.

The alignment check mark 606 comprises the first segment 661 and the second segment 662 so that the receiver 603 may be aligned with the wearer's urethral region particularly by using the second target as a guide. More specifically, the care personnel may place his or her finger on the second segment 662 and press the finger against the wearer's urethral region through the receiver 603 to confirm the wearer's urethral region to align the second segment 662 of the mark 606 with the wearer's urethral region. The alignment check mark 606 is positioned between the paired electrodes 645, 645 and assures it to position the wearer's urethral region in alignment with the position just between the paired electrodes 645, 645.
The first segment 661 of the mark 606 extends in the longitudinal direction Y and makes it possible to determine whether the receiver 603 is displaced or not over a wide range in the longitudinal direction Y. Considering that the urethral region is generally located along the longitudinal center line of the wearer's body, for example, the first segment may be aligned with the navel in the front waist region 621 and may be aligned with the bottom cleavage in the rear waist region 622 to align the intermediate region between the electrodes 645, 645 generally with the longitudinal center line of the wearer's body and thereby with the wearer's urethral region.

As has previously been described, it is possible after the article has been put on the wearer's body to determine the relative position between the longitudinal center line A-A of the receiver 603 and the wearer's body. Therefore, it is possible to correct the displacement of the receiver 603, if it has been determined. In this way, it is possible to avoid the problem that the moisture detecting sensor 641 of the moisture detecting structure 604 might be out of alignment with the wearer's urethral region and discharged urine could not be detected.

While the wearer's article is provided with the moisture detecting sensor 641 serving for detection of urine according to the present embodiment, it is possible to incorporate, in addition to the moisture detecting sensor 641, a feces detecting senor. While the mesh pants are used as the chassis 602 according to the present embodiment, it is also possible to use widely used means such as disposable diapers or diaper covers as the chassis 602.

While the alignment check mark 606 is formed only on the receiver 603 according to the present embodiment, it is possible to form the mark 606 on the chassis 602 also. In this case, the chassis 602 may be formed with the mark 606 extending in the longitudinal direction Y along the longitudinal center line of the chassis 602 and thereby the position of the chassis 602 can be adjusted so that the mark on the chassis 602 may be aligned with the mark on the receiver 603. Generally, the chassis 602 is scarcely displaced and the receiver 602 may be positioned in alignment with the mark of the chassis 602 to assure that the receiver 603 is positioned on the center line of the wearer's body.

While the second segment 662 of the mark 606 is positioned on the side of the front waist region 621 relatively to the transverse center line B-B according to the present embodiment, it is not essential. For example, the position of the second segment 662 may be appropriately changed depending on various factors such as the size of the wearing article or the receiver 603 or gender of the wearer. It should be appreciated that the second segment is not essential to be formed and, in contrast, two or more second segments may be formed, if desired.

### <Second Embodiment>

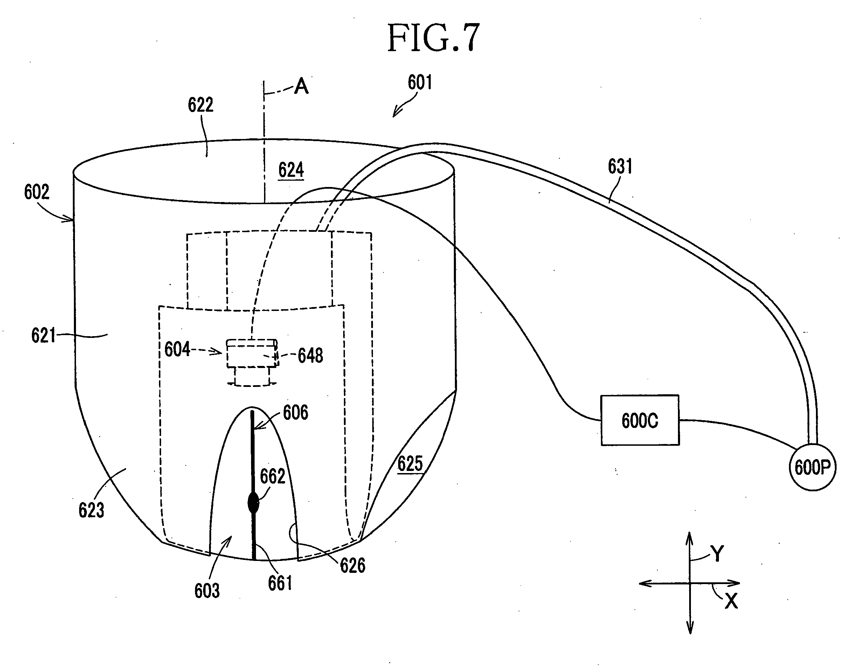

Fig. 7 is a diagram illustrating a second embodiment of the present invention. As will be seen in Fig. 7, the chassis 602 is formed in the crotch region 623 with a window 626 in which the receiver 603 is exposed. The window 626 allows the alignment check mark 606 formed on the receiver 603 to be visually recognized even if the chassis 602 is made of material having no see-through properties.

It is also possible to form the chassis 602 as a whole by material having no see-through properties and to form the crotch region 623 only by materials having see-through properties. For example, the chassis 602 may be formed of an opaque nonwoven fabric made of thermoplastic synthetic resin and a desired region of this nonwoven fabric may be heated under pressure to make it filmy and see-through. Alternatively, the chassis 602 may be formed of an opaque film made of thermoplastic synthetic resin containing inorganic fillers and a desired region of this film may be heated under pressure to smooth such region, to reduce optical diffuse reflection and thereby to make the region see-through. In this case, this film may be laminated together with the nonwoven fabric to reinforce this film or to provide the outer surface with a fabric-like touch.

Whatever the case may be, displacement of the receiver 603 relative to the wearer's urethral region can be checked and thereby it is assured that occurrence of urination can be detected.

## Claims

1. A wearing article having a longitudinal direction and a transverse direction, comprising a side facing the wearer's body and a side facing the wearer's garment, a chassis (602) having a front waist region (621), a rear waist region (622) and a crotch region (623) extending between said front and rear waist regions and a urine receiver (603) adapted to receive bodily fluids discharged onto said chassis, wherein:
a moisture detecting structure (604) is formed so as to extend along the longitudinal center line of said receiver,
wherein a pair of electrodes (645, 645) are formed in said moisture detecting structure (604) on both sides of the longitudinal center line of said receiver (603),
said wearing article being **characterized in that**: said receiver (603) is formed with an alignment check mark (606) which is positioned between said pair of electrodes (645, 645) and is adapted to be visually recognized from the outside through said chassis (602).

2. The wearing article according to claim 1, wherein said receiver has a longitudinal center line bisecting a length dimension in the transverse direction and said alignment check mark linearly extends in the longitudinal direction along the longitudinal center line.

3. The wearing article according to claim 1 or 2, wherein said alignment check mark comprises a first segment linearly extending along the longitudinal center line and a second segment having a length dimension in the transverse direction larger than that of said first segment.

4. The wearing article according to any one of claims 1 through 3, wherein, at least in said crotch region, said receiver is visually recognized through said chassis.

5. The wearing article according to any one of claims 1 through 4, wherein at least in said crotch region, said chassis is formed with windows through which the receiver is exposed.

## Patentansprüche

1. Kleidungsstück, das eine längslaufende Richtung und eine querlaufende Richtung hat, das Folgendes umfasst: eine Seite, die auf den Körper des Trägers hinzeigt, und eine Seite, das auf den Kleidungsstück des Trägers hinzeigt, einen Rahmen (602), der einen vorderen Taillenbereich (621), einen hinteren Taillenbereich (622) und einen Schrittbereich (623) hat, die sich zwischen den vorderen und hinteren Taillenbereichen erstrecken, und einen Urinfänger (603), der so adaptiert ist, dass er Körperflüssigkeiten auffängt, die an den Rahmen abgegeben werden, wobei
eine die Feuchtigkeit feststellende Struktur (604), die so geformt ist, dass sie sich an der längslaufenden Mittellinie des Auffängers entlang erstreckt,
wobei ein Elektrodenpaar (645, 645) in der die Feuchtigkeit feststellenden Struktur (604) auf beiden Seiten des längslaufenden Mittellinie des Empfängers (603) geformt ist,
wobei das Kleidungsstück **dadurch gekennzeichnet ist, dass** der Empfänger (603) mit einer Ausrichtmarkierung (606) geformt ist, die zwischen dem Elektrodenpaar (645, 645) positioniert und so adaptiert ist, dass es von der Außenseite durch den Rahmen (602) sichtbar erkannt wird.

2. Kleidungsstück nach Anspruch 1, wobei der Empfänger eine längslaufende Mittellinie hat, die eine Längendimension in Querrichtung halbiert, und sich die Ausrichtmarkierung in Längsrichtung an der längslaufenden Mittellinie entlang erstreckt.

3. Kleidungsstück nach Anspruch 1 oder 2, wobei die Ausrichtmarkierung ein erstes Segment, das sich linear an der längslaufenden Mittellinie erstreckt, und ein zweites Segment, das eine Längendimension in Querrichtung hat, die größer ist als die des ersten Segments, umfasst.

4. Kleidungsstück nach einem der Ansprüche 1 bis 3, wobei wenigstens im Schrittbereich der Empfänger durch den Rahmen sichtbar erkannt wird.

5. Kleidungsstück nach einem der Ansprüche 1 bis 4, wobei wenigstens im Schrittbereich der Rahmen mit Fenstern geformt wird, durch die der Empfänger freigelegt wird

## Revendications

1. Article vestimentaire ayant une direction allant dans le sens longitudinal et une direction allant dans le sens transversal, comportant un côté orienté vers le corps de l'utilisateur et un côté orienté vers le vêtement de l'utilisateur, une armature (602) ayant une région avant au niveau de la taille (621), une région arrière au niveau de la taille (622) et une région au niveau de l'entrejambe (623) s'étendant entre lesdites régions avant et arrière au niveau de la taille et un récepteur d'urine (603) adapté pour recevoir des fluides organiques déchargés sur ladite armature, dans lequel :
une structure de détection d'humidité (604) est formée de manière à s'étendre le long de la ligne centrale longitudinale dudit récepteur,
dans lequel une paire d'électrodes (645, 645) sont formées dans ladite structure de détection d'humidité (604) des deux côtés de la ligne centrale longitudinale dudit récepteur (603),
ledit article vestimentaire étant **caractérisé en ce que** :
ledit récepteur (603) est formé avec une marque de vérification d'alignement (606) qui est positionnée entre ladite paire d'électrodes (645, 645) et est adaptée pour être visuellement reconnue de l'extérieur au travers de ladite armature (602).

2. Article vestimentaire selon la revendication 1, dans lequel ledit récepteur a une ligne centrale longitudinale croisant une dimension de longueur dans la direction allant dans le sens transversal et ladite marque de vérification d'alignement s'étend linéairement dans la direction allant dans le sens longitudinal le long de la ligne centrale longitudinale.

3. Article vestimentaire selon la revendication 1 ou la revendication 2, dans lequel ladite marque de vérification d'alignement comporte un premier segment s'étendant linéairement le long de la ligne centrale longitudinale et un deuxième segment ayant une dimension de longueur dans la direction allant dans le sens transversal supérieure par rapport à celle dudit premier segment.

4. Article vestimentaire selon l'une quelconque des revendications 1 à 3, dans lequel, au moins dans ladite région au niveau de l'entrejambe, ledit récepteur est visuellement reconnu au travers de ladite armature.

5. Article vestimentaire selon l'une quelconque des revendications 1 à 4, dans lequel, au moins dans ladite région au niveau de l'entrejambe, ladite armature est formée avec une fenêtre au travers de laquelle le récepteur est exposé.
